# EUROPEAN PATENT SPECIFICATION

(11) **EP 3 295 938 B1**
(45) Date of publication and mention of the grant of the patent: **02.09.2020**
(21) Application number: 16189535.4
(22) Date of filing: 19.09.2016
(51) Int. Cl.: A61K 31/08, A61P 39/04

(54) **USE OF TRIETHYL ORTHOACETATE IN THE TREATMENT OF METAL OVERLOAD DISEASES**
VERWENDUNG VON TRIETHYL-ORTHOACETAT BEI DER BEHANDLUNG VON METALLÜBERBELASTUNGSERKRANKUNGEN
UTILISATION D'ORTHOACÉTATE DE TRIÉTHYLE DANS LE TRAITEMENT DE MALADIES DE SURCHARGE EN MÉTAL

(43) Date of publication of application: 21.03.2018
(73) Proprietor: ÜSKÜDAR ÜNIVERSITESI, 34662 Istanbul (TR); Sabanci Üniversitesi, 34956 Istanbul (TR)
(72) Inventor: ATASEVER ARSLAN, Belkis, 34662 Istanbul (TR); YILANCIOGLU, Kaan, 34662 Istanbul (TR); CETINER, Selim, 34956 Istanbul (TR); TIMUCIN, Ahmet Can, 34956 Istanbul (TR)
(74) Representative: Sevinç, Erkan

(56) References cited:
- SAGARA TAKEFUMI ET AL: "Non-protein components ofArthrospira(Spirulina)platensisprotect PC12 cells against iron-evoked neurotoxic injury", JOURNAL OF APPLIED PHYCOLOGY, KLUWER, DORDRECHT, NL, vol. 27, no. 2, 8 August 2014 (2014-08-08) , pages 849-855, XP035477847, ISSN: 0921-8971, DOI: 10.1007/S10811-014-0388-1 [retrieved on 2014-08-08]
- WU QINGHUA ET AL: "The antioxidant, immunomodulatory, and anti-inflammatory activities of Spirulina: an overview", ARCHIVES OF TOXICOLOGY, SPRINGER, DE, vol. 90, no. 8, 3 June 2016 (2016-06-03), pages 1817-1840, XP035999685, ISSN: 0340-5761, DOI: 10.1007/S00204-016-1744-5 [retrieved on 2016-06-03]
- PRIYANKA TYAGI ET AL: "THERAPEUTIC ADVANCEMENTS IN MANAGEMENT OF IRON OVERLOAD-A REVIEW Review Article", INTERNATIONAL JOURNAL OF PHARMACY AND PHARMACEUTICAL SCIENCES, vol. 7, no. 8, 15 July 2015 (2015-07-15), pages 35-44, XP055355742,
- SWARAN J.S. FLORA ET AL: "Chelation in Metal Intoxication", INTERNATIONAL JOURNAL OF ENVIRONMENTAL RESEARCH AND PUBLIC HEALTH, vol. 7, no. 12, 28 June 2010 (2010-06-28), pages 2745-2788, XP055010833, ISSN: 1661-7827, DOI: 10.3390/ijerph7072745

## Description

The present invention relates to the use of triethyl orthoacetate for reducing the excess of metal ions in subjects. In particular, the present invention is directed to use of triethyl orthoacetate for reducing the excess and toxic levels of iron stored in the body and in the organs.

### Background of the Invention

Metal ions are essential and play an important role in ensuring the proper functions of the living systems. Metal ions such as Fe³⁺, Al³⁺, Cd³⁺, Cr³⁺, Zn²⁺, Ca²⁺ and Cu²⁺ participate in the control of numerous metabolic and signalling pathways. Unfortunately, the body of a subject cannot absorb and excrete the total amount of metal ions; eventually excess metal in the body may result in toxicity. Specifically, accumulation of heavy metals results in serious health problems.

Iron is present in all cells and carries out vital functions. As an example, haemoglobin form of iron carries oxygen from the lungs to the tissues. It is stored intracellularly as ferritin; when the level of ferritin is more than the storage capacity, active iron is released intracellularly in the form of hemosiderin. Excess amount of iron can be toxic due to the reaction between iron and reactive oxygen species such as hydrogen peroxide. The iron reduces hydrogen peroxide and produces very reactive species, named hydroxyl radicals; this process is known as Fenton reaction. Hydroxyl radicals can attack the deoxyribose DNA backbone and bases, potentially causing a plethora of lesions that can be cytotoxic or mutagenic. The excess amount of absorbed iron is retained in the body and accumulates throughout the life-time. Excess accumulation of iron results in tissue damage and increased disease risks.

If iron levels are not regulated properly, it can become toxic by catalysing redox reactions, thereby resulting in the formation of free radicals which cause cell death especially in the liver, heart and endocrine glands as well as organ failure, and lead to death. Both genetic and non-genetic factors may cause excess of iron. Genetic conditions include various types of hemochromatosis, aceruloplasminemia or congenital atransferrinemia. Non-genetic causes of iron excess include dietary iron overload, transfusional iron overload, hemodialysis, chronic liver disease, porphyria cutanea tarda, post-portacaval shunting, dysmetabolic overload syndrome, iron tablet overdose, or any other cause of acute or chronic iron overload.

There have been various treatments used in the disorders caused by the excess of metal ions.

"Sagara T et al, 2015" is focused on polyphenolic compounds of A. platensis which are suggested to be active substances responsible for the protective effect of Arthrospira extract against the iron-evoked neurotoxicity and possibly for the protection of neurodegenerative disorders induced by excessive iron accumulation in the brain. Accordingly, an aqueous extract containing the non-protein components of A. platensis was prepared, and the activity of said extract on iron-evoked neurotoxicity was examined by determining the viability of PC12 cells in culture.

Iron chelation therapy is provided by the active ingredients involving deferiprone, deferoxamine and deferasirox.

Deferiprone is an orally active iron-chelating drug to be used in transfusional iron overload. Even though oral administration of deferiprone increases the patient compliance, it is associated with significant toxicity. Deferiprone is also associated with safety issues involving genotoxicity, neutropenia and agranulocytosis.

Other chelator, deferasirox is an effective and well-tolerated oral iron chelator elevating the adherence to iron chelating therapy among patients with iron overload. The U.S. Food and Drug Administration issued a warning about the potential adverse events associated with deferasirox in 2010. Acute renal failure, gastrointestinal bleeding and also acute liver necrosis can be seen in the patients who are administered with deferasirox.

Deferoxamine is poorly absorbed from the gastrointestinal tract; thus it is administered parenterally. Further, deferoxamine has short vascular half-life, about 5-10 minutes after intravenous infusion. In order to success a decrease in the iron levels, along duration of infusion is required. It is usually administered as a continuous subcutaneous infusion over 12-hour period, from three to seven times a week. Frequent intramuscular injections and continuous infusion result in poor patient adherence. Moreover, deferoxamine treatment is not much preferred due to its high cost and its adverse effects associated with hypotension, urticaria, and visual dysfunction.

Despite the benefits of therapies in which deferiprone, deferasirox or deferoxamine is administered, there are many drawbacks involving safety, toxicity, poor patient compliance and complexity of the regime.

The present invention aims to provide an effective method for treating metal overload disorders, specifically iron disorders; which is easily administered and safe, has minimized side effects and improved therapeutic efficiency.

Therefore, a need still exists for a novel, long-lasting, active metal-chelators with reduced toxicity and adverse effect which can be used in metal chelation therapy.

The present inventors conducted various experiments in order to evaluate iron chelating potentials of Spirulina platensis algae extract; which is then found to be effective as iron chelator. Compounds which are predominantly present in Spirulina Platensis are methyl stearate, methyl palmitate, methyl linoleate, thiosemicarbazide, triethyl orthoacetate and γ-methyl-linoleate.

The present inventors deepened the research and investigated the iron chelating activity of said compounds. Methyl palmitate and thiosemicarbazide complex with ferric ion, exhibiting constantly increasing RMSD values. Thus, methyl palmitate and thiosemicarbazide are considered to be ineffective due to the increasing RMSD values.

RMSD; means "root mean square displacement" and indicates the measure of the average distance between the atoms of the elements. Lower RMSD represents lower distance between the atoms, thus improved chelation activity.

Remaining compounds which comprise methyl linoleate, γ-methyl linoleate, methyl stearate and triethyl orthoacetate also complex with ferric ion and these compounds have adequate stability. However, triethyl orthoacetate becomes prominent since it remains in close proximity to ferric ion and has lower RMSD values; thereby having higher stability compared to other compounds.

Based on the extensive studies, triethyl orthoacetate is said to be advantageous as iron chelator.

### Summary of the Invention

The present invention is directed to the use of triethyl orthoacetate in the treatment of metal overload disorders.

The present invention also relates to a pharmaceutical composition that comprises triethyl orthoacetate for use in the treatment of metal overload disorders.

### Brief Description of the Figures

Accompanying figures are given solely for the purpose of describing the iron chelator activity of trietyhl orthoacetate in detail.
**Fig. 1** demonstrates the iron chelates with defaroxamine, methyl stearate, methyl palmitate, methyl linoleate, γ-methyl-linoleate, thiosemicarbazide and triethyl orthoacetate, prepared for molecular dynamics simulations.
**Fig. 2** demonstrates RMSD value variation of back bone atoms of all molecules according to simulation time.
**Fig. 3** demonstrates the measurement of the distance between center of mass of molecules to the center of mass of ferric iron.
**Fig. 4** demonstrates the distance distribution vs. Distance graph of the distances shown in Fig. 3.
**Fig. 5** demonstrates the snapshot of deferoxamine-ferric iron complex at 0 ns and 3 ns.
**Fig. 6** demonstrates the snapshot of triethyl orthoacetate-ferric iron complex at 0 ns and 3 ns.
**Fig. 7** demonstrates the iron chelating activity of pure triethyl orthoacetate at 100, 200 and 400 mM concentration respectively.

### Detailed Description of the Invention

Triethyl orthoacetate is represented by the structural formula (I) as described below.

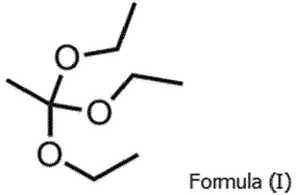

Triethyl orthoacetate (1,1,1-triethoxyethane) is used in pharmaceutical applications, in particularly as solvent.

It has now been found that trietyhl orthoacetate has satisfactory pharmacological properties when used in the treatment of metal overload disorders which are caused by excess of a heavy metal, particularly excess of iron. Triethyl orthoacetate is efficient in chelating and removing iron from the body, it possesses suitable bioavailability, and has eliminated side effects with minimal toxicity to the subject.

"Heavy metals" indicates any metals and metalloid that are toxic for the organism even at low concentrations. Many of the heavy metals, such as zinc, copper, chromium, iron and manganese, are essential to body function in very small amounts. But, if these metals accumulate in the body in concentrations that lead to poisoning, then serious damage may occur. The heavy metals most commonly associated with poisoning of humans are lead, mercury, arsenic and cadmium.

In accordance with the present invention, triethyl orthoacetate is used for the treatment of heavy metal overload disorders. It is considered to be within the scope of the invention that heavy metal elements include iron, aluminium, chromium, copper, zinc, manganese, mercury, arsenic and cadmium, and in particular iron.

Within the scope of the present invention, "treatment of heavy metal overload disorder" means the control of the heavy metal ion levels in a subject.

Triethyl orthoacetate is used in an amount effective to achieve the intended purpose; it has the advantage of possessing high iron clearing efficiency and improved oral bioavailability.

"Iron clearing efficiency" refers to the effectiveness of triethyl orthoacetate in excreting iron from the body or one of the organs thereof. This also concerns the amount of iron removed from the target area in unit time. Thus, effective amounts of triethyl orthoacetate not only remove iron from the body of the subject, but also provide a serious decrease of iron-induced damage to the organs.

Subjects suffering from a condition caused by excess of metal ion in the body can be treated with a therapeutically effective amount of triethyl orthoacetate.

In an embodiment of the invention, the metal overload that may be treated by triethyl orthoacetate according to the present invention is iron overload.

According to the invention, iron overload is associated with thalassemias, hemochromatosis type 1 (classical hemochromatosis), hemochromatosis type 2A or 2B (juvenile hemochromatosis), hemochromatosis type 3, hemochromatosis type 4 (African iron overload), neonatal hemochromatosis, aceruloplasminemia, congenital atransferrinemia, dietary iron overload, transfusional iron overload, hemodialysis, chronic liver disease, hepatitis C, cirrhosis, non-alcoholic steatohepatitis, porphyria cutaneatarda, post- portacaval shunting, dysmetabolic overload syndrome, iron tablet overdose, cancer diseases, concomitant disorders (e.g. severe anaemia, hypoproteinaemia, renal or cardiac failure) preclude phlebotomy, iron storage diseases, sideroblastic anaemia, auto-immune haemolytic anaemia, chronic anaemias, Pantothenate Kinase-Associated Neurodegeneration (PKAN), Wilson disease or any combination thereof.

The term "iron overload disorder" refers to a condition in a subject, preferably human, wherein the level of iron in the body is elevated compared to the level of iron in a healthy subject.

Elevated iron levels may be measured by using a calorimetric assay, the standard transferrin saturation assay or the standard serum ferritin assay.

Trietyhlorthoacetate is used for the treatment of iron overload disorders in subjects comprising all mammals, preferably humans.

Daily administration of an amount of triethyl orthoacetate is in the range of 10-100 mg/kg of body weight of the subject, and preferably in the range of 10-40 mg/kg of body weight of the subject. The dose of triethyl orthoacetate provides therapeutic benefit without causing substantial toxicity.

The optimal dose is adjusted in accordance with the following parameters; the iron overload disorder to be treated, severity of iron overloading, the age of the subject, the weight of the subject, sex of the subject, renal and hepatic function of the subject, the mode of administration, and the like. Thanks to the use of correct daily dosage of triethyl orthoacetate, iron damages due to the elevated levels of iron can be successfully prevented.

Therapeutically effective amount means an amount for preventing or ameliorating the symptoms or prolonging the life time of the subject being treated.

In some embodiments, the invention can be used in a subject who has a serum ferritin level greater than about 1,000 ng/ml.

Triethyl orthoacetate is administered to the subject until the serum ferritin reaches to a value of about 1,000 ng/ml or below and the treatment is resumed as soon as the serum ferritin level exceeds of about 1,000 ng/ml.

The term subject means all mammals including humans.

"Treating" or "treatment" as used herein, covers the treatment of an iron disorder in a subject, preferably human, or a disease associated with an iron disorder in a subject, preferably human, includes: (i) preventing an iron disorder in said subject or a disease associated with an iron disorder in said subject, (ii) inhibiting an iron disorder in a subject, or a disease associated with an iron disorder the subject, (iii) relieving an iron disorder in a subject or a disease associated with an iron disorder in the subject, (iv) relieving symptoms of an iron disorder in a subject or a disease associated with iron disorder in the subject, (v) restoring/maintaining the normal serum iron levels in a subject having an iron disorder or having a disease associated with an iron disorder.

The present invention relates also to pharmaceutical compositions comprising triethyl orthoacetate.

According to an embodiment, pharmaceutical compositions comprising triethyl orthoacetate is used for the treatment of metal overload disorders which are caused by heavy metals. Metal overload disorder is preferably iron overload disorder.

In an embodiment, the present invention provides pharmaceutical compositions comprising triethyl orthoacetate together with at least one pharmaceutical acceptable excipient.

The pharmaceutical composition of the invention may contain a pharmaceutically acceptable excipient selected from a buffer, a preservative, a surfactant, a lubricant, a glidant, a diluent, binder, disintegrant, stabilizer, wetting agent, emulsifier and combinations thereof.

Triethyl orthoacetate may be present in amount between 1mg and 1000 mg per unit dose.

The pharmaceutical compositions may contain from about 0,1% to about 99%, preferably from about 10% to about 50% of triethyl orthoacetate by weight.

Pharmaceutical compositions comprising triethyl orthoacetate may be administered one, two, three or more times daily depending on the daily dosage.

Pharmaceutical compositions of triethyl orthoacetate may be administered in a manner such as intravenously, subcutaneously, transdermally, transmucosally, sublingually, rectally, orally or buccally, in a preferred embodiment pharmaceutical compositions of triethyl orthoacetate is administered orally or subcutaneously.

In another embodiment of the invention, the pharmaceutical composition according to the invention is a solid dosage form, solution or suspension formulation. Solid dosage form may be formulated as powder, granule, chewing gum, pill, tablet or capsule. The preferred dosage form is powder, tablet or capsule.

The daily intake of trietyhl orthoacetate may be in the range of 1 mg - 2000 mg for the treatment of iron overload disorders.

Pharmaceutical compositions of triethyl orthoacetate may be in the form of an immediate release tablet, a controlled release tablet, a sustained release tablet, a modified release tablet, a disintegrating tablet or a delayed release tablet.

In another embodiment of the invention, the pharmaceutical composition of the invention has immediate release profile. The release of the pharmaceutical compositions may also be in controlled manner.

Pharmaceutical compositions comprising triethyl orthoacetate may be manufactured by means of conventional processes such as mixing, granulating and compressing methods.

The present invention will now be illustrated by the following Examples, which are not intended to be limiting in any way.

### EXAMPLES

### Example-1:

### Iron chelating activity of Spirulina Platensis extract

Iron chelating potential of Spirulina Platensis algae extract is analysed at 5000 µg/mL, 2000 µg/mL and 1000 µg/mL concentrations. EDTA solution is chosen as positive control agent because of its iron binding capacity. Methanol is used as negative control.

According to the results, high chelating activity of Spirulina Platensis demonstrated at 5000 µg/mL and 2000 µg/mL concentrations.

**Table 1: Iron chelating activity of Spirulina Platensis**

| *Concentration of Spirulina Piatentsis extract (µg*/*mL)* | *Iron chelating activity (%)* |
|---|---|
| 5000 | 76.2 |
| 2000 | 54.8 |

### Example-2:

### Identification of Contents of Spirulina Extract

The content of Spirulina Platensis extract is analysed by GS-MS method. 42 compounds are identified in the extract. Major compounds are specified as follows;

**Table 2: The content of Spirulina Platensis**

| **Compound** | **Amount (%)** |
|---|---|
| Methyl stearate | 39.87 |
| Methyl palmitate | 19.61 |
| Methyllinoleate | 7.18 |
| Thiosemicarbazide | 4.40 |
| Triethyl orthoacetate | 4.11 |
| γ-Methyl-linoleat | 3.36 |
| Octadecanoic acid | 3.04 |

### Example-3:

### Evaluation of iron chelating activity of triethyl orthoacetate in silico and in vitro

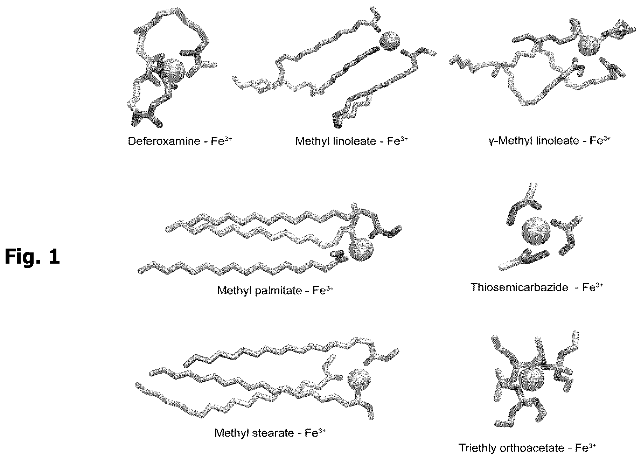

All of the complexes comprising methyl stearate, methyl palmitate, methyl linoleate, thiosemicarbazide, triethyl orthoacetate and γ-methyl-linoleate complex with ferric ion as seen in Fig. 1.

Methyl palmitate and thiosemicarbazide complexed with ferric iron displayed constantly increasing RMSD values and eliminated from analysis at that step.

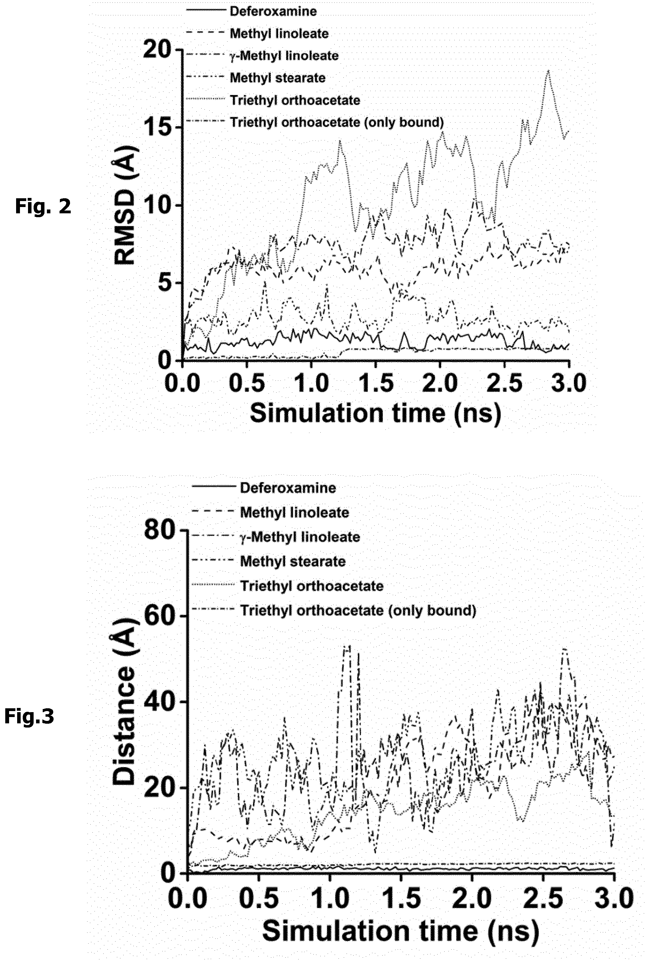

Moreover, according to Fig. 2 rest of the complexes reached a plateau of RMSD values indicating stable structures for further analysis. Deferoxamine is positive control agent in this experiment.

In particular, triethyl orthoacetate that remained in close proximity to ferric iron (short dash dotted black line) displayed even lower RMSD values, thus higher stability compared to its whole simulation (dotted black line).

As seen in Fig. 3; distance from the center of mass of the single triethyl orthoacetate molecule to the center of mass of the ferric iron showed very similar distance values to the positive control, deferoxamine-ferric iron complex during 3 ns of MD simulations (solid black line vs.short dash dotted black line).

The distribution vs distance analysis of all distances presented that triethyl orthoacetate-ferric iron complex and positive control had similar distance distribution and displayed much lower values than other simulations (solid black line and short dash dotted black line vs. others).

Representative snapshot of triethyl orthoacetate and ferric iron at the end of 3ns were also very similar to the deferoxamine-ferric iron complex in terms of proximity of each molecule to ferric iron.

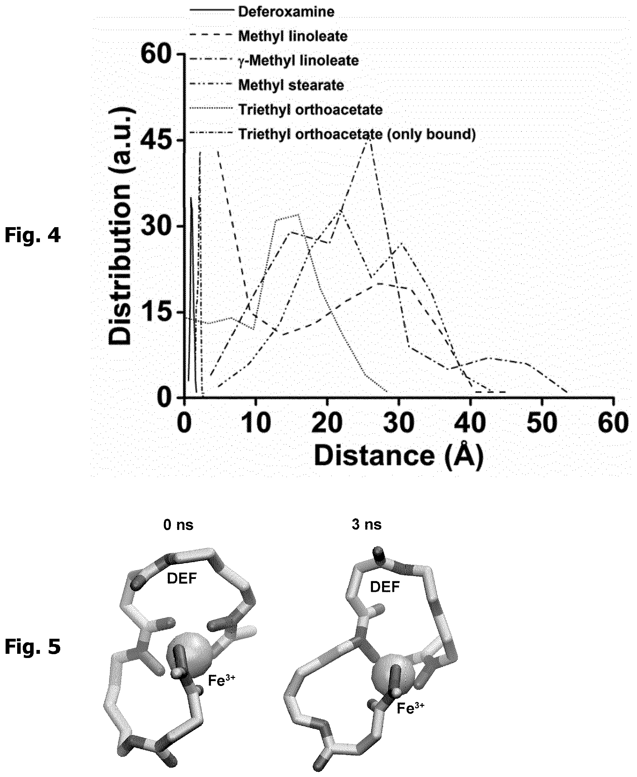

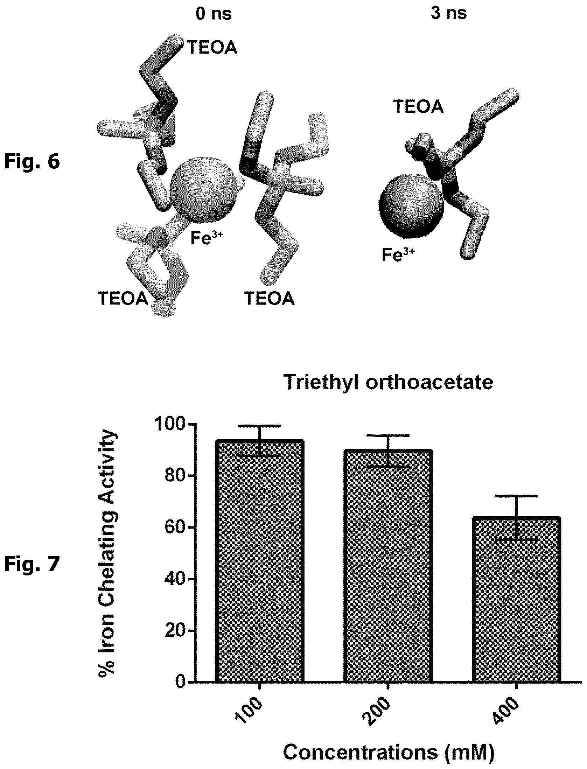

In Figure 5 and 6, before and after complexes are shown in simulation analyses representatively.

In silico analysis mentioned above in detail, demonstrates that triethyl orthoacetate has potential chelating activity on iron. Furthermore, in vitro experiments with pure triethyl orthoacetate compound was conducted in order to testify the simulated chelating activity shown in computational analysis. As seen in Fig. 7, 100, 200 and 400 mM pure triethyl orthoacetate has been shown to suggest strong in vitro iron chelating activity over %70 in a parallel manner of computational simulation results demonstrated previously.

In Fig. 2-6; DEF refers to deferoxamine, TEOA refers to triethyl orthoacetate, Fe³⁺ refers to ferric iron and Å refers to Angstrom.

### Example-4:

### In-vivo and in-vitro studies

### 1. Algae culture and extraction protocol:

Spirulina platensis was cultivated inZarrouk's Medium medium. Instructions for preparing the culture media were retrieved from the University of Texas at Austin (UTEX) culture collection of algae (http://www.sbs.utexas.edu/utex/mediaDetail.aspx?mediaID=166).

Spirulina platensis was extracted by using pure methanol prior to bead-beater homogenization. Methanolic extract was evaporated by a rotavapor and was further dissolved in methanol using an ultrasonic bath, and filtered just before the cytotoxicity assays.

### 2. GS-MS analysis:

Content was analyzed using a Shimadzu gas chromatograph (QP5050, NY, USA), equipped with a Rtx®-5MS column (30 mx0.25 mm ID, 0.10 µm film thickness). Nitrogen was used as a carrier gas (average flow rate, 1 ml/min). Oven temperature program was from 110°C (2 min) to 200°C at 10°C/min, then to 300°C at 5°C/min, 9 min post run at 300°C. Injector and detector (FID, Quadrupole(MS)) temperatures were 250°C and 280°C, respectively. The MS working in electron impact mode at 70 eV; ion source temperature 200°C; mass spectra data were acquired in the scan mode in m/z range 45-450.

### 3. Identification of methanolic content:

Content were identified by using National Institute Standard and Technology (NIST) library (http://www.nist.gov/srd/nistla.cfm). Component relative percentages were calculated based on GC peak areas without using correction factors.

### 4. Iron chelation activity of Spirulina platensis extract and pure triethyl orthoacetate:

Spirulina platensis extracts were prepared at 5000µg/ml, 2000µg/ml, 1000µg/ml concentrations. As a positive control, 2.5 mM EDTA solution was used because of its high iron binding capacity. As a negative control only methanol was used. Negative controls and methanolicmicroalgae extracts (400 µl) were pipetted into 24 well plate, then 100 µl of 0.25mM FeSO4 solution (dissolved in methanol) was added. For the reaction initiation, 100 µl 5mM ferrozine dissolved in methanol was added. Solution was homogenized by pipetting and incubated for 10 minutes at room temperature. After incubation, it was analysed by using spectrophotometric measurement at 562nm wavelength. In vitro experiments with pure triethyl orthoacetate compound was also conducted with the concentrations of 100, 200 and 400 mM respectively. Same experimental protocol was used for pure triethyl orthoacetate as described in detail above for Spirulina platensis extract.

### 5. Molecular dynamics simulations for predicting a candidate chelator:

Six different molecules were used for predicting the most plausible candidate chelator; Methyl linoleate, γ-Methyl linoleate, Methyl stearate, Methyl palmitate, Thiosemicarbazide and Triethyl orthoacetate. Deferoxamine was used as positive control. Initial configurations were prepared in SPARTAN program using three of each molecule and a ferric iron ion. Ferric iron coordinates for deferoxamine were adapted from PDB ID: 1K2V. Each structure was optimized through semiempirical PM6 method in GAUSSIAN 09 quantum chemistry program and visualized using GaussView 5.0 program. Parameter and topology for each structure were prepared using Swiss-Param and corrected according to the gaussian output. Molecular dynamics (MD) simulations were implemented using seven complex structures. As the positive control, deferoxamine-ferric iron complex was used in MD simulations. All complexes, composed in between ∼10000 to ∼16000 atoms were placed in water boxes with dimensions of 40x40x40 Å³ respectively. Then all systems were ionized to a neutral state. The resulting systems were used in MD simulations using the NAMD program with the CHARMM22 parameters which included correction map (CMAP) for backbone atoms. Water molecules within the system were treated explicitly using the TIP3P model. An NpT ensemble was used in MD simulations with periodic boundary conditions, and the long-range Coulomb interactions were computed using the particle-mesh Ewald algorithm. Pressure was maintained at 1 atm and temperature was maintained at 310 K using the Langevin pressure and temperature coupling. A time step of 2 fs was used in all MD simulations. The systems were fully energy minimized in 20,000 steps and carefully equilibrated under constant temperature and volume for 0.5 ns. Then they were heated slowly from 10 K to 310 K in 30 ps before production runs. The production were lasted for 3 ns and repeated twice. Visual molecular dynamics (VMD) was used for the analysis of trajectories and the visualization of structures. Root mean square displacements (RMSD) were calculated for all atoms of each molecule excluding hydrogens. RMSD for the single triethyl orthoacetate in close proximity to ferric iron at the end of 3 ns, was also calculated for clear representation of the main finding.

## Claims

1. A compound of triethylorthoacetate for use in the treatment of iron overload disorders.

2. The compound for use according to claim 1, wherein the iron overload is associated with thalassemias, hemochromatosis type 1 (classical hemochromatosis), hemochromatosis type 2A or 2B (juvenile hemochromatosis), hemochromatosis type 3, hemochromatosis type 4 (African iron overload), neonatal hemochromatosis, aceruloplasminemia, congenital atransferrinemia, dietary iron overload, transfusional iron overload, hemodialysis, chronic liver disease, hepatitis C, cirrhosis, non-alcoholic steatohepatitis, porphyria cutaneatarda, post-portacaval shunting, dysmetabolic overload syndrome, iron tablet overdose, cancer diseases, concomitant disorders (e.g. severe anaemia, hypoproteinaemia, renal or cardiac failure) preclude phlebotomy, iron storage diseases, sideroblastic anaemia, auto-immune haemolytic anaemia, chronic anaemias, Pantothenate Kinase-Associated Neurodegeneration (PKAN), Wilson disease, or any combination thereof.

3. The compound for use according to claim 1, wherein triethyl orthoacetate is administered to subjects comprising mammals, preferably humans.

4. The compound for use according to claim 1, wherein the daily dosage of triethyl orthoacetate is between 10-100 mg/kg of body weight of the subject and preferably 10-40 mg/kg of body weight of the subject.

5. The compound for use according to claim 4, wherein the serum ferritin level of the subject is greater than about 1000 ng/L.

6. A pharmaceutical composition that comprises triethyl orthoacetate for use in the treatment of iron overload disorders.

7. The pharmaceutical composition for use according to claim 6, wherein said composition comprises at least one excipient.

8. The pharmaceutical composition for use according to claim 6, wherein triethyl orthoacetate is present in amount between 1mg and 1000 mg per unit dose.

9. The pharmaceutical composition for use according to claim 6, wherein said composition is administered orally.

10. The pharmaceutical composition for use according to claim 9, wherein said composition is in the solid dosage form.

11. The pharmaceutical composition for use according to claim 10, wherein said composition is in the form of a tablet.

12. The pharmaceutical composition for use according to claim 11, wherein said composition is an immediate release tablet, controlled release tablet, sustained release tablet, modified release tablet, disintegrating tablet or delayed release tablet.

## Patentansprüche

1. Eine Verbindung von Triethylorthoazetat zur Verwendung bei der Behandlung von Eisenspeicherkrankheit.

2. Verbindung zur Verwendung nach Anspruch 1,
wobei die Eisenspeicherkrankheit assoziiert ist mit Eisenspeicherkrankheit mit Thalassämien, Hämochromatose Typ 1 (klassische Hämochromatose), Hämochromatose Typ 2A oder 2B (juvenile Hämochromatose), Hämochromatose Typ 3, Hämochromatose Typ 4 (afrikanische Eisenspeicherkrankheit), neonataler Hämochromatose, Aceruloplasminämie, kongenitale Atransferrinämie, diätetische Eisenspeicherkrankheit, transfusionsbedingte Eisenspeicherkrankheit, Hämodialyse, chronische Lebererkrankung, Hepatitis C, Zirrhose, nicht alkoholische Steatohepatitis, Porphyria cutaneatarda, post-portakavales Shunting, dysmetabolisches Überlastungssyndrom, Eisentablettenüberdosierung, Krebserkrankungen, Begleiterkrankungen (z.B. schwere Anämie, Hypoproteinämie, Nieren- oder Herzinsuffizienz) schließen Aderlass, Eisenspeicherkrankheiten, sideroblastische Anämie, autoimmune hämolytische Anämie, chronische Anämien, Pantothenatkinase-assoziierte Neurodegeneration (PKAN), Morbus Wilson oder jede Kombination davon aus.

3. Verbindung zur Verwendung nach Anspruch 1, wobei Triethylorthoazetat an Subjekte verabreicht wird, die Säugetiere, bevorzugt Menschen, umfassen.

4. Verbindung zur Verwendung nach Anspruch 1, wobei die tägliche Dosis von Triethylorthoazetat zwischen 10 bis 100 mg/kg Körpergewicht des Lebewesens und bevorzugt zwischen 10 bis 40 mg/kg Körpergewicht des Lebewesens liegt.

5. Verbindung zur Verwendung nach Anspruch 4, wobei der Serumferritinspiegel des Lebewesens größer als etwa 1.000 ng/L ist.

6. Pharmazeutische Verbindung, die Triethylorthoazetat aufweist, zur Verwendung bei der Behandlung von Eisenspeicherkrankheit.

7. Pharmazeutische Verbindung zur Verwendung nach Anspruch 6, wobei die Verbindung zumindest einen Hilfsstoff enthält.

8. Pharmazeutische Verbindung zur Verwendung nach Anspruch 6, wobei Triethylorthoazetat in einer Menge zwischen 1 mg und 100 mg pro Dosiseinheit vorhanden ist.

9. Pharmazeutische Zusammensetzung zur Verwendung nach Anspruch 6, wobei die Zusammensetzung oral verabreicht wird.

10. Pharmazeutische Zusammensetzung zur Verwendung nach Anspruch 9, wobei die Zusammensetzung in fester Dosierungsform vorliegt.

11. Pharmazeutische Zusammensetzung zur Verwendung nach Anspruch 10, wobei die Zusammensetzung in Form einer Tablette vorliegt.

12. Pharmazeutische Zusammensetzung zur Verwendung nach Anspruch 11, wobei die Zusammensetzung eine Tablette mit sofortiger Freisetzung, eine Tablette mit kontrollierter Freisetzung, eine Tablette mit verlängerter Freisetzung, eine Tablette mit modifizierter Freisetzung, eine zerfallende Tablette oder eine Tablette mit verzögerter Freisetzung ist.

## Revendications

1. Composé d'orthoacétate de triéthyle pour une utilisation dans le traitement de troubles liés à une surcharge en fer.

2. Composé pour l'utilisation selon la revendication 1, dans lequel la surcharge en fer est associée à des thalassémies, à une hémochromatose de type 1 (hémochromatose classique), à une hémochromatose de type 2A ou 2B (hémochromatose juvénile), à une hémochromatose de type 3, à une hémochromatose de type 4 (surcharge en fer africaine), à une hémochromatose néonatale, à une acéruloplasminémie, à une atransferrinémie congénitale, à une surcharge alimentaire en fer, à une surcharge en fer transfusionnelle, à une hémodialyse, à une maladie hépatique chronique, à une hépatite C, à une cirrhose, à une stéatohépatite non alcoolique, à une porphyrie cutanea tarda, à un shunt post-porto-cave, à un syndrome de surcharge dysmétabolique, à une surdose de comprimés de fer, à des maladies cancéreuses, à des troubles concomitants (par exemple une anémie sévère, une hypoprotéinémie, une insuffisance rénale ou cardiaque) empêchant la phlébotomie, à des maladies liées au stockage du fer, à une anémie sidéroblastique, à une anémie hémolytique auto-immune, à des anémies chroniques, à une neurodégénérescence associée à un déficit en pantothénate kinase (PKAN), à une maladie de Wilson ou toute combinaison de ceux-ci.

3. Composé pour l'utilisation selon la revendication 1, dans lequel l'orthoacétate de triéthyle est administré à des sujets comprenant des mammifères, de préférence des êtres humains.

4. Composé pour l'utilisation selon la revendication 1, dans lequel la posologie quotidienne d'orthoacétate de triéthyle est comprise entre 10 et 100 mg/kg de poids corporel du sujet et de préférence entre 10 et 40 mg/kg de poids corporel du sujet.

5. Composé pour l'utilisation selon la revendication 4, dans lequel le taux de ferritine sérique du sujet est supérieur à environ 1000 ng/L.

6. Composition pharmaceutique comprenant de l'orthoacétate de triéthyle pour une utilisation dans le traitement de troubles liés à une surcharge en fer.

7. Composition pharmaceutique pour l'utilisation selon la revendication 6, dans laquelle ladite composition comprend au moins un excipient.

8. Composition pharmaceutique pour l'utilisation selon la revendication 6, dans laquelle l'orthoacétate de triéthyle est présent en une quantité comprise entre 1 mg et 1000 mg par dose unitaire.

9. Composition pharmaceutique pour l'utilisation selon la revendication 6, dans laquelle ladite composition est administrée par voie orale.

10. Composition pharmaceutique pour l'utilisation selon la revendication 9, dans laquelle ladite composition est sous la forme posologique solide.

11. Composition pharmaceutique pour l'utilisation selon la revendication 10, dans laquelle ladite composition est sous la forme d'un comprimé.

12. Composition pharmaceutique pour l'utilisation selon la revendication 11, dans laquelle ladite composition est un comprimé à libération immédiate, un comprimé à libération contrôlée, un comprimé à libération prolongée, un comprimé à libération modifiée, un comprimé à délitement ou un comprimé à libération retardée.
